# EUROPEAN PATENT APPLICATION

(11) **EP 0 557 511 A1**
(43) Date of publication of application: **01.09.1993**
(21) Application number: 92920347.9
(22) Date of filing: 15.09.1992
(51) Int. Cl.: A61M 5/32

(54) **SIMPLIFIED SECURITY SYRINGE**

(30) Priority: 18.09.1991 ES 910206
(71) Applicant: JESGAMABE, S.L., E-46002 Valencia (ES)
(72) Inventor: GALIANA SABATER, Amando, E-46001 Valencia (ES)
(74) Representative: Sanz-Bermell Martinez, Alejandro
(86) International application number: ES9200069
(87) International publication number: WO9305833

(57) **Abstract**

The invention relates to a security syringe having a conventional cylinder (1), a stopper (2) connected to a pusher rod provided with an extension (3) susceptible of engagement into a part (9) which supports the needle (8) having a female portion joined to the rear envolving part which supports the needle, or made in a single piece with a weakening (35) susceptible of breaking. It has two annular flanges (11, 13) for interlocking the seal (10) and a weakening portion (12) for breaking the stopper. Application to the fabrication of syringes.

## Description

The subject of the present invention is a safety syringe whose main feature is the fact that being a safety syringe, it has the same number of parts and these parts are assembled in the same way and manner as the traditional syringes without the safety features stated below.

Traditional syringes are manufactured by parts, i.e., bodies or cylinders, pistons and plungers in one or in two pieces, and needles. Safety syringes examined below are normally made up of more pieces. Its larger number of pieces depends on the conditions under which the mentioned safety condition is established.

It is clear that syringes that offer security are up to now complex; they are also more expensive than the simple ones and in addition to these their use has some inconveniences. And it is also evident that simple syringes did not have enough safety features. Therefore the present alternative is to pay a lot of money for each disposable syringe, or use traditional ones at the already known price.

The fact that the removable needle/needle holder is an essential condition is also to be considered because syringes with a fixed needle/needle holder cannot be used to carry out a liquid extraction from a glass capsule or through a rubber cover of the capsule and then inject the patient with the same needle. It is also to be taken into account that if the extraction of the liquid is carried out with the same needle, the sensitivity and softness of the material the needle is made of may be damaged with the rubbing of the glass of the capsule and even with the rubber of the cover which will produce tearing on the patient. Therefore they are not good for the aforesaid uses.

The aim of the present syringe is to make accidents impossible after its use, due to the introduction of the needle in its body; In addition to this, its conditions allow production at the same price level as the syringes with traditional features. Moreover, the manufacturing of this syringe is far cheaper than the manufacturing of any of the existing ones included in the technological repertory described below. It also has the advantage of being universal; It can be produced in the insulin type, ordinary type and it can also be valid for veterinary use, as manufacturing conditions, means and assemblies are identical in all of them. This patent contains two essential types of production: The first type in which it is formed by a cone shaped harpoon and a lodging in the needle formed by a piece manufactured separately from the holder and conveniently inserted; and a second type of manufacturing, more complex as far as its mould is concerned but cheaper because it suppresses assemblies, made up of a cylindrical piston head and a lodging in the needle made up of a single pressure/traction breakable piece manufactured in one single process with its holder.

The known technical precedents of this patent are:
ES-P 8802473, invented by the applicant of this syringe in collaboration with others, consisting of a syringe formed by a body with an outer cylinder, an inner casing with openings, through which slides a Greek-letter "omega" shaped inner piece, whose forward prolongation has a harpoon shaped end and has an all-around joint that constitutes the piston of the syringe. The needle can have its support separated from the needle. The functioning of this needle is established in such a way that the forward prolongation of the "omega"-shaped piece, the harpoon end, is inserted in the inner part of the needle so that the retraction of the plunger implies the extraction of the needle, separating the "omega"-shaped piece from the piston and being trapped on its way back by the openings on the inner cover. The manufacturing of this syringe, that has too many pieces, is not viable with traditional costs, and the new patent considerably improves the manufacturing costs as well as its simplicity.

US-4 507 117 consisting on a syringe formed by a cylinder which has a needle inside attached to a piece that is detachable from the bottom of the cylinder and assembled to the piston. Its main disadvantage is that the needle is fixed. This needle is inserted through the inside of the cylinder and its assembling requires great accuracy. The fact that the needle is not removable makes its use impossible except for extractions. It cannot have a general use. The new patent is as simple but allows the extraction of the liquid with a needle, the changing of the needle for a new one with which the patient can be injected and the destruction by extraction to the inside of the cylinder,of the needle that has been in contact with the patient's blood
US-4 631 057, consisting of a syringe which has a double cylinder whose inner sides determine the cavity that houses the liquid to be injected, and the outer sides determine the piece that can be moved along the cylinder with an anti-return device at the end of the stroke when this outer cylinder is trapped by an appropriate latch. This syringe is more expensive than the one proposed because of the number of pieces and of the mounting difficulty. In addition to these, it is not as safe because a finger can not touch the tip end of the needle and get infected by it.

US-4 692 156, consisting of a syringe that has in the forward part of the sealing joint a female device that can be assembled to a male device joint to the needle which is fixed. This syringe is mounted from the rear part by a highly accurate machine that makes the manufacturing process more expensive, and it is not good for general use and, due to the fixed needle, it can only be used for extractions which makes its use non profitable.

EP-0 326 983 A2 which consist in a syringe with very similar features to the aforesaid US-4 692 156. The difference is that it uses a male device at the end of the piston with the female device located in the rear part of the needle which remains, as the previous one mentioned, inside the cylinder, with a meticulous and expensive mounting. The needle cannot be changed in any case due to its structure and therefore it is only valid for blood extractions, and therefore the new patent we propose has higher efficiency conditions.

US-4 747 830 which consists of a very complex syringe that has a piston formed by a double sealing piece which has at its rear end two legs to avoid the extraction of the piston and whose forward end has a piece that is shaped to match the piece at the rear of the needle which is inside the syringe and is not removable. This syringe, as the ones mentioned before, has a great manufacturing difficulty due to its complexity as well as its assembling, in addition to the fact that the needle is fixed and cannot be changed that makes it valid only for blood extractions.

PCT/AU 88/00441 consisting of a syringe that has a piston ending in a male device, of complex profile, that assembles with the rear part of the fixed needle and that is introduced through the rear of the cylinder of the syringe. It has the same features as the above mentioned patents and the disadvantage compared with the present patent is that the needle is fixed and therefore only valid for extractions.

It is to be pointed out that the above mentioned patents with fixed needle, although valid for some uses, are not suitable for general use. This prevents them from being manufactured alternatively with others of a more appropriate type. Consequently, it is easier, cheaper, more profitable and creates fewer problems of storing, ordering, etc., if all syringes, no matter what their use, are equal.

In order to make the following explanation clearer and more understandable, four pages of drawings are enclosed with eleven figures representing, as an example, the essence of the present invention.

Figure 1 shows a schematic view of the syringe in load/unload position.

Figure 2 shows the syringe in the final unload/needle coupling position.

Figure 3 shows the syringe in the position of needle extracted.

Figure 4 shows the piston of the syringe.

Figure 5 shows the outer side of the syringe.

Figure 6 shows a general view of the syringe, needle, piston and body.

Figure 7 shows the outer piece that holds the needle. Figure 8 shows the inner part that supports the needle.

Figure 9 shows a detailed view of the rear locking mechanism of the piston head.

Figure 10 shows the piston head and its prolongation in detail.

Figure 11 shows in detail the head carrying the needle and forming one body with the detachable inner part.

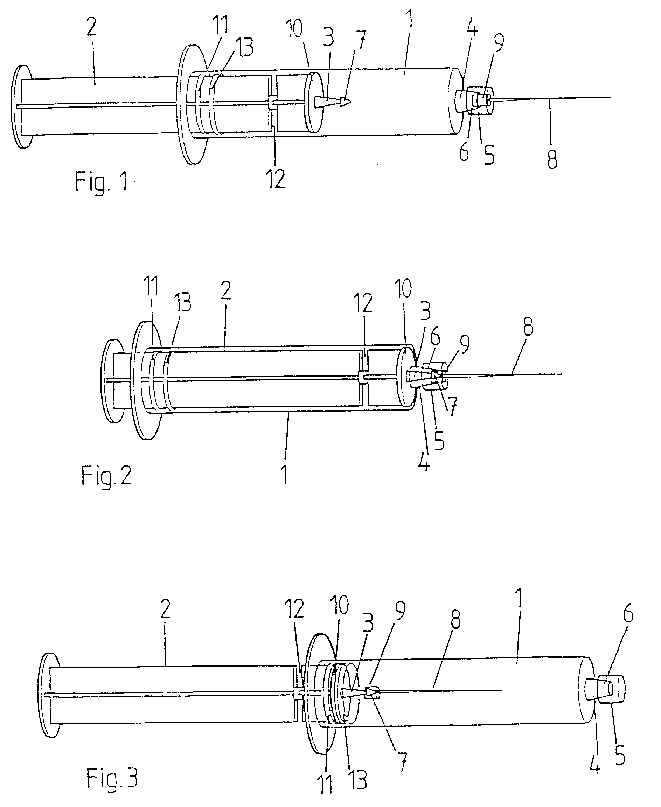

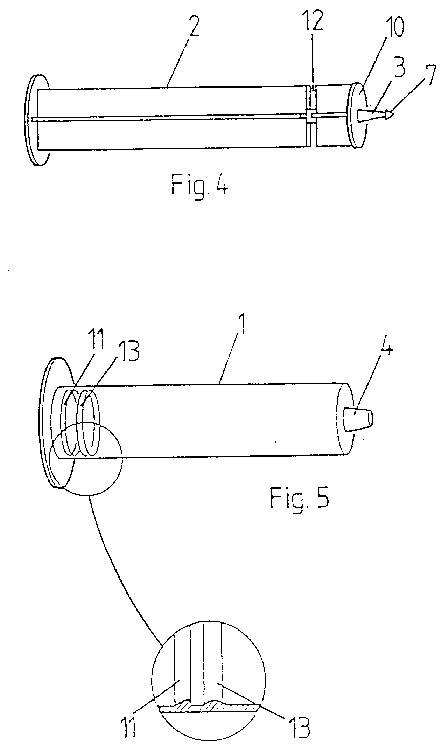

Figure 1 shows a schematic view of the syringe in load/unload position where the outer cylinder 1, with identical general features of the known ones, can be appreciated. The only visible difference from the others is the detail at the end portion that can clearly be seen in figure 5. The forward slightly cone shaped end can have a larger diameter than the traditional one, not too relevant, just the measurement required to facilitate the way for the piston prolongation 3. The piston 2 formed by a body with a sealing joint 10 at the end can also be appreciated. This joint is made of the same material as the piston itself, producing the sealing adjustment by pressure on the walls of cylinder 1 and its adjustment is perfect due to the fact that the walls are elastic facilitating the adaptation of the mentioned joint. The piston prolongation 3 is part of piece 2, as well as the joint 10. Said piece 2 is formed by injection and is unique, indivisible and does not have welding or joints. Number 4 indicates the forward end of the cylinder which forms a tube at whose end 6 the needle set will be inserted. The said needle is represented here by an enveloping rear piece 5, aimed at being assembled by pressure in part 6 of end 4. The needle 8 is held by cohesion to piece 5, that has a complementary shape to end 7 inside which it will assemble with the rear base 9 of the needle 8. The piston part marked 12 has a weak point in order to produce its breakage by bending the piston once its use is finished. At the rear end the piston has two inwardly prominent ring-shaped nerves 11 and 13 which are described in detail in figure 5.

Figure 2 shows the syringe in the final unload/needle coupling position in which parts and pieces mentioned in the previous figure are appreciated. In this position piece 3 is introduced in end 4 of the cylinder whose end 7, of special shape, is introduced in piece 9 which is inseparably united to the needle 8. This syringe produces fewer empty spaces than traditional ones due to its prolongation 3, and therefore makes better use of the injectable liquid.

Figure 3 shows the syringe in needle extracted position. In this figure it is appreciated that end 7 of piece 3 is attached to the rear base 9 of the needle so that with a further pull on of piston 2 the introduction of the needle inside the cylinder is achieved.

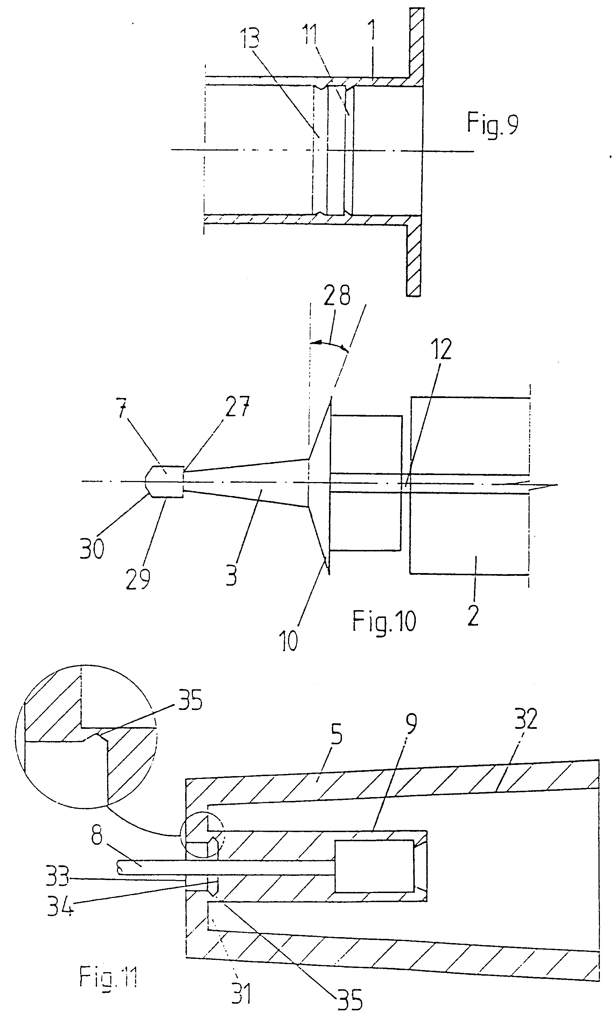

Figure 4 shows the piston of the syringe and the form of the sealing joint 10, and the disposition in one single piece of said body with its forward prolongation 3 and its end 7. one can also appreciate the weak point 12 of the piston at the appropriate point to produce its breakage once the needle is introduced and the joint 10 is between the two ledges 11 and 13 in figures 5 and 9.

Figure 5 shows the outer part of the syringe where 1 is the body and 11 and 13 are the ring shaped nerves of the cylinder that, when the piston is extracted, will hook the part of the piston which constitutes the sealing joint marked 10 in figures 1 to 4. The inner ring shaped nerve 13 has an asymmetric form allowing the joint 10 to pass by with some difficulty. The ring shaped nerve 11 has a shape that determines the possibility of sliding towards the inside and because of its staggered inner form prevents the extraction of the sealing joint of the piston that remains hooked here in the breaking position of the body of the piston.

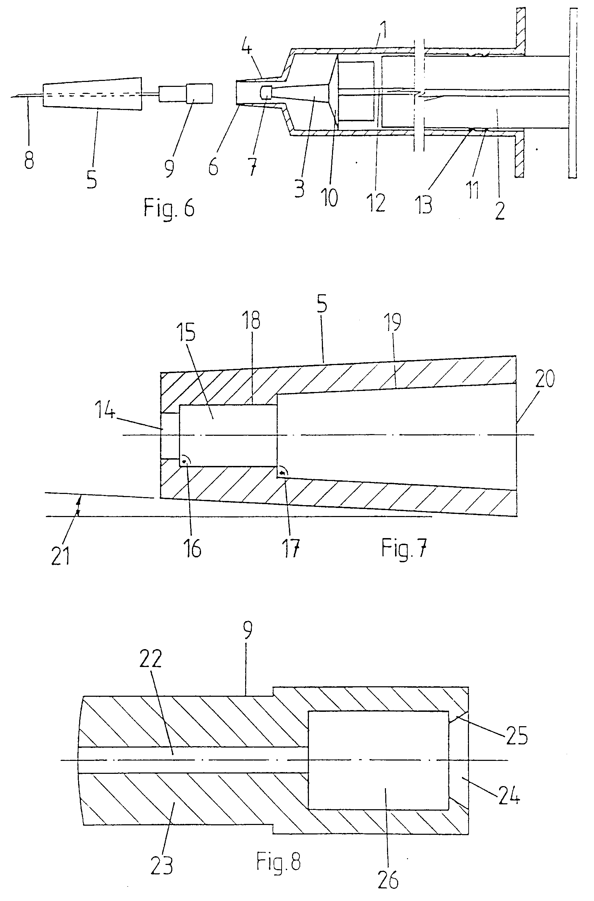

Figure 6 shows a view of the whole syringe, needle, piston and body, where a specific detail of this syringe can be appreciated at the end of the piston 7 where there is a structure with higher resistance to pressure / traction due to a specific form which will be analyzed later on. The base of the needle 9 has a form which determines a simpler assembling on the rear enveloping piece of the needle 5. The sealing joint 10 is very thin with a sharp contour and exerts a sharp pressure on the outer cylinder, and therefore it achieves a high degree of sealing and an easy transit along the cylinder.

Figure 7 shows a view of the outer piece that is separated from the needle holder where 14 is the hole for the needle, 15 is the lodging for the insertion of the rear base of the needle 9 formed by a cylindrical structure with parallel walls 18 and square angles 16. Number 17 indicates the slightly obtuse angles 17 that determine the origin of rear inner walls of truncated cone shaped generatrix; 20 is the rear inlet that is adjustable to the forward end of the cylinder 4 of figures 1 to 6, and 21 is the acute angle that determines the conicity of the walls of piece 5.

Figure 8 shows a view of the inner piece 9 that holds the needle and where 22 is the hollow cylinder for the lodging and blocking of the needle; 23 is the forward part of the rear base of the needle 9 which has an identical size as its lodging 15 in figure 7, being totally cylindrical and having the inlet 24 for the end 7 and being 25 the narrowing that allows the entrance of said end 7 by elasticity and stops the extraction of said end 7 once it has been totally introduced.

This layout, although it has great advantages over its known precedents, is a little more expensive to produce than traditional syringe needles. This is due to the fact that the base of the needle has two pieces 5-9 that have to be assembled and this assembling involves a cost.

In order to avoid manufacturing costs of two different pieces and its later assembling, figure 11 describes a single piece that is identical, as far as manufacturing costs are concerned, to the known needle bases, that gathers pieces 5 and 9 together, is detachable and requires no assembling.

Figure 9 shows a detailed view of the locking mechanism of the sealing joint 10 at the end portion of the cylinder that is formed by a ring shaped nerve 13, shaped prominently inwards. The sealing joint 10 can lodge between this ring and the second nerve 11 also ring shaped but with a triangular shape with the prominent angle inwards that determines the impossibility of backward movement in relation to the piercing shape of the sealing joint 10 in figures 6 and 10.

Figure 10 shows a detailed view of the head of the piston and its prolongation where the specific shape of the piston prolongation 3 whose forward end 7, that has its side walls 29 shaped like a cylinder of revolution with parallel walls, is linked to piston 3 with its rear part forming an 90° angle 27 that is free due to the smaller diameter of the prolongation of piston 3 than the one of the cylinder shaped end 7. The forward end of the cylinder shaped end in this figure, is made up of a facetted form 30 whose section has a flat frontal surface and two sides of facets slightly sloped though not piercing. 28 shows the angle of gradient between the joint profile 10 and the perpendicular of the piston axis, having a higher gradient than the wall on which it rests inside the end of the cylinder so that to extract the needle the piston is lightly pressed determining the possibility of clasping angle 27 in lodging 26 of figure 8 because of the angling of both pieces, piston and inner forward wall of the cylinder.

Figure 11 shows a detailed view of the needle holder 5 formed by a single body with detachable inner part 9 whose inner cavity continues up to the forward inner wall 31, the side inner walls being slightly cone shaped. The needle 8 is placed from the forward end. The length of rear base of the needle 9 can be shorter according to the cohesion required with the mentioned needle. The needle is fixed through the needle access hole 33 which determines an emptying of material that cooperates to debilitate the fitting of the rear base of the needle 9. The rear base of the needle 9 has a forward wall 34 whose contour united to the body 5 has a weak point 35 in the link point of the two elements in order to enable the breakage by pressure/traction of this piece. Piece 9 in this figure, except for the link to the rear enveloping piece 5, has a shape identical to the parts marked 24, 25, 26 in figure 8. The resistance to pressure/traction of the piston end 7 of figure 10, and of the narrowing 25 of inlet 24 of piece 9, must be bigger than that at the weak point marked 35.

The present syringe works in the same way as traditional ones. It is loaded through a needle 8 and it produces the extraction by piston 2, determining inside the cylinder a space where the liquid is housed. Then the excess air is expelled, and needle changed if necessary before introducing it into the patient's body. After the injection has been carried out, the piston must be pressed in such a way that, according to figure 2, the end 7 of the piston prolongation 3 is inserted inside the needle, which means that a new extraction of the piston determines the introduction of the needle in the cylinder. Then, as a safety measure, piston 2 can be broken in order to prevent further use of the syringe or the needle sticking out again, otherwise the syringe is also made unusable but occupies more volume.

For the use of the syringe as a means of blood extraction, like the traditional ones, the needle is introduced into the vein with the piston near its end position and the extraction is produced in the ordinary way. Once the blood is put into the glasses available in these clinics, the introduction of the needle is produced as explained in the previous paragraph.

A preferred make of this patent will be formed by the outer cylinder with its ring shaped edges located as per figure 9, the piston explained in figure 10 and the needle whose holder is configured according to the unique piece representation in figure 11.

## Claims

1. Simplified safety syringe, formed by cylinder piston and needle, comprising a cylinder (1) of the traditional type, characterised by the fact that the piston(2) is formed by one single piece which has at its forward end a sealing joint (10) and a prolongation (3) and which at its rear end has a stem or plunger with a weak point (12) near the forward end, the nozzle, cylindrical-cone shaped at its forward end (4), being of small diameter and enabling prolongation (3) of piston (2) to get through and be inserted by its external perimeter to the base or rear end of the needle holder (5) which is initially linked and undetachable from a part (9) or rear base of the needle which is attached or forms a piece that can be detached by traction, pressure or breakage with the holder of said needle (8) and with the above mentioned base (5) being able to be fixed and detached from the end (6) of the cylinder by elastic pressure fixation, and with piece (9) that supports the needle (8) having a female shape that can be assembled to the male cone or cylindrical shaped end (7) of the prolongation (3), which determines of the separation by traction of the support (5) of the piece (9) that holds the needle.

2. Syringe, according to claim 1, characterised in that its prolongation (3) is at the forward part of the piston, before the sealing joint (10), injected in one single piece at least with said joint, and preferably with the inner whole, piston-male prolongation-plunger stem, and having this prolongation a certain longitudinal conicity.

3. Syringe, according to claim 1, characterised in that at the rear end of the cylinder it has two narrowings formed by two ring-shaped nerves, having the inner ring (13) asymmetric section and the outer ring (11) asymmetric triangular section determining in its inner part the shape of a little step.

4. Syringe, according to claim 1, characterised in that the sealing joint (10) is formed by a ring that forms one single piece with the whole piston (2), the contour of this ring being very acute and forming an angle of gradient (28) with respect to the perpendicular of the axis of the piston; this gradient must be bigger than the one of the wall of the inner end of the cylinder on which it rests, determining an empty space at the end of its run that can be occupied by a certain pressure on the plunger.

5. Syringe, according to claim 1, characterised in that the end (7) has cylindrical form as far as its side walls that are parallel, and is linked to the piston prolongation (3) determining at its rear part an angle of 90° that has its contour free due to the smaller diameter of the prolongation of the piston (3) than that of the cylinder shaped end (7). The forward end of the cylinder shaped end in this figure, is made up of a facetted form (30) whose section has a flat frontal surface and two sides of facets slightly sloped though not piercing.

6. Syringe, according to claim 1, characterised in that the outer piece that holds the needle (5) and whose rear base (9) can be separated, is formed by a body with trunco-conical structure which has a forward hole through which the needle (8) passes, a lodging (15) with parallel cylindrical walls (18) with right angles (16), and a second rear space of slightly trunco-conical generatrix (19) that has its origin in the slightly oblique angles (17) and continues up to its rear end which has an inlet (20) that can be coupled with the end (6) of the cylinder (1).

7. Syringe, according to claims 1 and 6, characterised in that the rear base (9) of the needle (8) is formed by a body that has a cylindrical hole (22) for lodging the needle (8) made up of a cylindrical forward part (23) of the same diameter as its lodging (15), and a rear part that constitutes a lodging space for the piston end (7) determined by an inlet suitably narrowed by a ring shaped rim with some elasticity 25.

8. Syringe, according to claim 1, 6 and 7, characterised in that the needle holder head formed by one single piece (5-9) is made up of a body that is essentially trunco-conical at its outer part in which an inner hole continues up to the forward inner wall (31) and having both inner side walls (32) slightly conical, placing the needle (8) in the forward part fixed by a needle access hole (33) that determines the emptying of material that cooperates in the weakening of the fixation of the rear base (9) of the needle which has a forward wall (34) whose contour united to the body (5) has a weak point (35) made in the joint of both detachable elements according to the possibility of breaking it by pressure / traction on this piece which determines the separation of the outer body (5) and inner one (9), the resistance to pressure / traction of the end of the piston (7) and the narrowing (25) being bigger than that at the ring shaped weak point (35).
